# EUROPÄISCHE PATENTSCHRIFT

(11) **EP 3 128 325 B1**
(45) Veröffentlichungstag und Bekanntmachung des Hinweises auf die Patenterteilung: **02.01.2019**
(21) Anmeldenummer: 15186743.9
(22) Anmeldetag: 24.09.2015
(51) Int. Cl.: G01N 33/569

(54) **VERFAHREN ZUR BESTIMMUNG EINER KONZENTRATION EPITHELIALER ZELLEN IN EINER BLUTPROBE ODER ASPIRATSPROBE**
METHOD FOR DETERMINING A CONCENTRATION OF EPITHELIAL CELLS IN A BLOOD SAMPLE OR ASPIRATE SAMPLE
PROCEDE DE DETERMINATION D'UNE CONCENTRATION DE CELLULES EPITHELIALES DANS UN ECHANTILLON DE SANG OU D'ASPIRAT

(30) Priorität: 07.08.2015 EP 15180229; 10.09.2015 EP 15184733
(43) Veröffentlichungstag der Anmeldung: 08.02.2017
(73) Patentinhaber: Pachmann, Ulrich, 95448 Bayreuth (DE); Pachmann, Katharina, 95448 Bayreuth (DE)
(72) Erfinder: Pachmann, Ulrich, 95448 Bayreuth (DE); Pachmann, Katharina, 95448 Bayreuth (DE)
(74) Vertreter: Dr. Gassner & Partner mbB

(56) Entgegenhaltungen:
- WO-A1-2014/020169
- MONIKA PIZON ET AL: "Insulin-Like Growth Factor Receptor I (IGF-IR) and Vascular Endothelial Growth Factor Receptor 2 (VEGFR-2) Are Expressed on the Circulating Epithelial Tumor Cells of Breast Cancer Patients", PLOS ONE, Bd. 8, Nr. 2, 13. Februar 2013 (2013-02-13), Seite e56836, XP055231459, DOI: 10.1371/journal.pone.0056836
- HEKIMIAN KATYA ET AL: "Demasking of epithelial cell adhesion molecule (EpCAM) on circulating epithelial tumor cells by Tween(R)20 treatment in breast cancer patients", CLINICAL CHEMISTRY AND LABORATORY MEDICINE,, Bd. 50, Nr. 4, 1. April 2012 (2012-04-01), Seiten 701-708, XP009187369,
- LIXIA ZHAO ET AL: "Comparison and development of two different solid phase chemiluminescence ELISA for the determination of albumin in urine", ANALYTICA CHIMICA ACTA, Bd. 541, Nr. 1-2, 1. Juni 2005 (2005-06-01), Seiten 197-205, XP055231531, NL ISSN: 0003-2670, DOI: 10.1016/j.aca.2005.03.027

## Beschreibung

Die Erfindung betrifft ein Verfahren zur Bestimmung einer Konzentration epithelialer Zellen in einer aus einem Menschen oder Säugetier stammenden und mit einem gerinnungshemmenden Mittel versetzten Blutprobe oder Aspiratsprobe. Bei der Aspiratsprobe kann es sich um eine Probe eines Knochenmarkaspirats, eines Pleuralaspirats oder eines Peritonealaspirats handeln.

Ein solches Verfahren ist aus der US 7,615,358 B2 bekannt. Dabei werden in einer Körperflüssigkeit enthaltene epitheliale Tumorzellen durch Zusatz von Antikörpern oder Antikörperfragmenten markiert, wobei die Antikörper oder Antikörperfragmente gegen das humane epitheliale Antigen gerichtet sind, das durch den monoklonalen Antikörper HEA 125 erkannt wird. Die Probe wird anschließend auf einen Träger aufgebracht und inkubiert, um die Tumorzellen an den Träger adhärieren zu lassen. Die Oberfläche des Trägers kann dabei mit einem die unspezifische Zellbindung unterstützenden Agens, wie z. B. Poly-L-Lysin, beschichtet sein. Die Adhäsion der Zellen dauert üblicherweise 10 bis 15 Minuten. Anschließend werden vitale Zellen der adhärierenden epithelialen Tumorzellen mittels ihrer Morphologie identifiziert und quantifiziert und die Konzentration vitaler epithelialer Tumorzellen in der Körperflüssigkeit berechnet. Dabei wird die Morphologie der adhärierenden epithelialen Tumorzellen mittels Laser-Scanning-Cytometrie analysiert, wobei lebende Tumorzellen durch ihre exklusive Oberflächenfärbung detektiert und tote Zellen auf Grund ihrer intrazellulären Färbung ausgeschlossen werden.

Aus der WO 2014/047285 A1 ist ein Verfahren zur Detektion und/oder Behandlung einer Untergruppe von Prostatakrebspatienten, die von einer Taxanbehandlung profitieren können, bekannt. Dabei wird geprüft, ob in einer von den Patienten entnommenen Probe eine Androgenrezeptor-Spleißvariante vorhanden ist. Dazu können aus der Probe zirkulierende Tumorzellen abgefangen und auf das Vorhandensein einer der spezifischen Spleißvarianten getestet werden. Das Abfangen kann durch immobilisierte Antikörper erfolgen. Das Testen umfasst es, die Probe mit einem gegen die Spleißvariante gerichteten Antikörper in Kontakt zu bringen und eine Bindung des Antikörpers an die Spleißvariante zu detektieren.

Aus Hekimian, K. et al., Clin. Chem. Lab. Med. 2012; 50(4), Seiten 701-708 ist eine Demaskierung epithelialer Zelladhäsionsmoleküle (EpCAM) auf in Brustkrebspatientinnen zirkulierenden epithelialen Tumorzellen durch Tween®20-Behandlung bekannt. In der Veröffentlichung wird spekuliert, dass EpCAM durch Glykoproteine oder Membran-Lipoproteine maskiert ist, so dass dadurch eine Antikörperbindung verhindert wird. In einem Experiment wurde jeweils 1 ml gerinnungsgehemmtes peripheres Blut jeweils am Tag der Entnahme und am ersten und zweiten Tag nach der Entnahme für 5 Minuten mit 20 µl Tween®20 oder ohne das Detergens inkubiert. Anschließend wurden die darin enthaltenen Erythrozyten mit einem Erythrozytenlysepuffer lysiert. Epitheliale Zellen wurden dann mit einem gegen EpCAM gerichteten Antikörper detektiert. Dabei zeigte sich, dass mit Tween®20-Behandlung am Tag der Entnahme, am Tag 1 und am Tag 2 nach der Entnahme eine im Rahmen der Fehlergrenzen gleich hohe Zellzahl detektiert werden konnte. Ohne Tween®20 konnten am Tag der Entnahme keine, am ersten Tag etwas weniger und am zweiten Tag gleich viele Zellen wie unter Tween®20-Behandlung detektiert werden. Dies zeigt, dass auch eine Lagerung der Blutproben vor der Inkubation mit den Antikörpern die Zugänglichkeit von EpCAM auf den Zellen für die Antikörper erhöht.

Aufgabe der vorliegenden Erfindung ist es, ein alternatives Verfahren zur Bestimmung einer Konzentration epithelialer Tumorzellen in einer aus einem Menschen oder Säugetier stammenden und mit einem gerinnungshemmenden Mittel versetzten Blutprobe oder Aspiratsprobe anzugeben.

Die Aufgabe wird durch die Merkmale des Patentanspruchs 1 gelöst. Zweckmäßige Ausgestaltungen ergeben sich aus den Merkmalen der Patentansprüche 2 bis 15.

Die Erfindung betrifft ein Verfahren zur Bestimmung einer Konzentration epithelialer Zellen in einer aus einem Menschen oder Säugetier stammenden und mit einem gerinnungshemmenden Mittel versetzten Blutprobe oder Aspiratsprobe. Dabei wird die Probe nach Zusatz von Antikörpern, Antikörperfragmenten oder Antikörpermimetika, die jeweils gegen ein für epitheliale Zellen spezifisches Antigen gerichtet sind, solange gelagert, bis sich die Zunahme der Zahl der an die Zellen gebundenen Antikörper, Antikörperfragmente oder Antikörpermimetika in Abhängigkeit von der Zeit verlangsamt, d. h. bis die Bindungsrate abnimmt bzw. bis eine durch Auftrag der Bindung gegenüber der Zeit erhaltene Bindungskurve beginnt abzuflachen und in den Bereich der Sättigung überzugehen. Erst dann wird die Anzahl der markierten Zellen und daraus die ursprüngliche Konzentration dieser Zellen in der Blutprobe oder Aspiratsprobe bestimmt. Es hat sich nämlich erstaunlicherweise gezeigt, dass das Erreichen des Beginns des Abflachens der Sättigungskurve sehr viel länger dauert als dies bei einer gegebenen Anzahl an Bindungspartnern zu erwarten wäre. Es wird vermutet, dass viele der für epitheliale Zellen spezifischen Antigene zunächst maskiert vorliegen und erst im Laufe der Zeit für eine Bindung der Antikörper, Antikörperfragmente oder Antikörpermimetika zugänglich werden. Erst dann ist eine zuverlässige Bestimmung der Zellzahl möglich.

Konkret umfasst das erfindungsgemäße Verfahren zur Bestimmung einer Konzentration epithelialer Zellen in einer aus einem Menschen oder Säugetier stammenden und mit einem gerinnungshemmenden Mittel versetzten Blutprobe oder Aspiratsprobe die folgenden Schritte:
a) Lysieren der in der Blutprobe oder Aspiratsprobe enthaltenen Erythrozyten durch Zugabe eines ein Lysieren der Erythrozyten bewirkenden Puffers, Absondern dabei nicht lysierter Zellen und Suspendieren der abgesonderten Zellen in einem Puffer wobei die Blutprobe oder die Aspiratsprobe für mindestens 24 Stunden bei einer Temperatur zwischen 0 und 40 °C gelagert wird, bevor die Erythrozyten lysiert werden,
b) Zugabe der je mindestens eine Markierung tragenden Antikörpern, Antikörperfragmenten oder Antikörpermimetika, die jeweils gegen das Epitheliale Zelladhäsionsmolekül (EpCAM) und/oder mindestens ein sonstiges für epitheliale Zellen spezifisches Antigen gerichtet sind, zu einer bei Schritt a) erhaltenen Zellsuspension oder einer durch Absondern erhaltenen Teilmenge dieser Zellsuspension, Vermischen der Antikörper, Antikörperfragmente oder Antikörpermimetika und der Zellsuspension oder der Teilmenge und Inkubation einer dadurch erhaltenen Mischung für mindestens eine Zeit, welche zum Erreichen einer abnehmenden Bindungsrate einer Bindung der Antikörper, Antikörperfragmente oder Antikörpermimetika an die Zellen erforderlich ist, wobei die Inkubation für mindestens 6 Stunden erfolgt,
c) Bestimmen der Anzahl von durch eine Bindung der Antikörper, Antikörperfragmente oder Antikörpermimetika markierten Zellen in der bei Schritt d) erhaltenen Mischung und
d) Berechnung der Konzentration der bei Schritt c) bestimmten Anzahl markierter Zellen in der Blutprobe oder Aspiratsprobe.

Die Durchführung des gesamten Verfahrens erfolgt außerhalb des menschlichen und außerhalb des tierischen Körpers. Das Vermischen der Antikörper, Antikörperfragmente oder Antikörpermimetika und der Teilmenge gemäß Schritt b) kann unmittelbar nach der Zugabe der Antikörper, Antikörperfragmente oder Antikörpermimetika zu der Teilmenge erfolgen.

Bei der Berechnung der Konzentration der bei Schritt c) bestimmten Anzahl markierter Zellen in der Blutprobe oder Aspiratsprobe gemäß Schritt d) ist eine gegebenenfalls vorhergehend erfolgte Verdünnung und/oder Konzentrationserhöhung der Zellen gegenüber der Blutprobe oder Aspiratsprobe zu berücksichtigen.

Normalerweise finden sich keine epithelialen Zellen im zirkulierenden Blut. Es hat sich jedoch gezeigt, dass sich im Blut von Patienten mit einem malignen epithelialen Tumor, wie z. B. einem Lungen-, Brust-, Darm- oder Prostatatumor, zirkulierende epitheliale Tumorzellen im Blut finden. Weiterhin hat es sich gezeigt, dass sich im Blut von Patienten mit chronischen inflammatorischen Erkrankungen eines epithelialen Gewebes, wie z. B. Rheumatoider Arthritis, Asthma, COPD, Morbus Crohn oder Colitis ulcerosa, epitheliale Zellen dieses Gewebes im peripheren Blut finden lassen. Darüber hinaus gelangen epitheliale Zellen durch epitheliales Gewebe schädigende Ereignisse, wie z. B. eine Operation oder eine Quetschung ins periphere Blut. Es ist daher zu Zwecken einer Diagnose einer Erkrankung an einem epithelialen Tumor oder einer chronischen inflammatorischen Erkrankung eines epithelialen Gewebes, zum Überwachen eines Krankheitsverlaufs oder Heilungsprozesses und für Vorsorgeuntersuchungen bzw. Massenscreenings nützlich, wenn die Konzentration epithelialer Zellen in einer aus einem Menschen oder Säugetier stammenden Blutprobe oder Aspiratsprobe möglichst genau bestimmt werden kann. Die Zellen können dabei Zellen eines epithelialen Tumors, epitheliale Zellen aus einem entzündeten Gewebe oder sonstige durch ein epitheliales Gewebe schädigendes Ereignis in den Blutkreislauf eingedrungene epitheliale Zellen sein.

Bei dem gerinnungshemmenden Mittel kann es sich z. B. um Ethylendiamintetraacetat (EDTA), Citrat oder Heparin handeln.

Bei den Antikörperfragmenten kann es sich beispielsweise um Fab', F(ab')₂ oder Fab handeln. Es kann sich dabei aber auch um rekombinante Antikörperfragmente handeln, beispielsweise scFv, di-scFv, sdAb (single domain anti body) oder um chemisch verbundene Antikörperfragmente wie F(ab')₂. Bei Antikörpermimetika handelt es sich um Verbindungen, die wie Antikörper zur Bindung von Antigenen befähigt sind, ohne jedoch selbst Antikörper oder Antikörperfragmete zu sein. Dabei handelt es sich zumeist um künstliche Peptide, Proteine oder Lektine.

Das Bestimmen der Menge gebundener Antikörper, Antikörperfragmente oder Antikörpermimetika gemäß Schritt c) erfolgt in Abhängigkeit von der Zeit ab dem Vermischen gemäß Schritt b). Dem Fachmann auf dem Gebiet der Untersuchung der Bindung von Antikörpern, Antikörperfragmenten oder Antikörpermimetika an Substrate ist es bekannt, wie eine Bindungsrate bestimmt wird.

Die Erfinder der vorliegenden Erfindung haben erkannt, dass die Bindung der markierten Antikörper, Antikörperfragmente oder Antikörpermimetika an ein für Epithelzellen spezifisches Antigen auf der Oberfläche der zirkulierenden epithelialen Zellen nicht, wie zu erwarten, in einem Zeitraum weniger Minuten nach deren Zugabe und Vermischung eine Sättigung erreicht, sondern dass eine Sättigung erst nach mehreren Stunden erreicht wird. Da dies nicht allein auf der Antigen-Antikörper-Reaktion beruhen kann, wird angenommen, dass es eine zunehmende Zugänglichkeit zuvor maskierter Epitope gibt. Die Zugänglichkeit wird bereits durch eine Lagerung vor Inkubation mit den Antikörpern, wie sie aus Hekimian, K. et al. bekannt ist, erhöht. Sie wird jedoch nochmals durch eine langanhaltende Inkubation in Gegenwart der Antikörper, Antikörperfragmente oder Antikörpermimetika erhöht. Ursache dafür könnte sein, dass durch deren Bindung eine Demaskierung der Antigene erreicht wird. Ein derartiger Effekt ist für den Fachmann völlig überraschend.

Zwischen den Schritten a) und b) können die folgenden weiteren Schritte durchgeführt werden:
a1) Absondern mindestens einer Teilmenge oder einer weiteren Teilmenge der bei Schritt a) erhaltenen Zellsuspension,
a2) Zugabe der je mindestens eine Markierung tragenden Antikörper, Antikörperfragmente oder Antikörpermimetika, die jeweils gegen das Epitheliale Zelladhäsionsmolekül (EpCAM) und/oder mindestens ein sonstiges für epitheliale Zellen spezifisches Antigen gerichtet sind, zu der weiteren Teilmenge in einer solchen Menge, dass deren Konzentration in der weiteren Teilmenge mit der Konzentration der Antikörper, Antikörperfragmente oder Antikörpermimetika in der Zellsuspension oder der Teilmenge gemäß Schritt b) identisch ist, Vermischen der Antikörper, Antikörperfragmente oder Antikörpermimetika und der weiteren Teilmenge und Inkubation einer dadurch erhaltenen Mischung bei einer vorgegebenen Temperatur und
a3) fortlaufendes oder mehrfaches Bestimmen einer Menge von Antikörpern, Antikörperfragmenten oder Antikörpermimetika, die an in der weiteren Teilmenge enthaltene Zellen in Abhängigkeit von der Zeit ab dem Vermischen gemäß Schritt a2) gebunden sind, ermitteln einer Bindungsrate daraus und Bestimmen einer Zeit, die zum Erreichen einer abnehmenden Bindungsrate der Bindung der Antikörper, Antikörperfragmente oder Antikörpermimetika an diese Zellen erforderlich ist,
wobei die Inkubation bei Schritt b) bei der vorgegebenen Temperatur erfolgt, wobei die Zeit gemäß Schritt b) so gewählt ist, dass sie zumindest so lange ist, wie die bei Schritt a3) bestimmte Zeit.

Bei der bei Schritt a3) zu bestimmenden Menge an Antikörpern, Antikörperfragmenten oder Antikörpermimetika kann es sich um eine relative oder eine absolute Menge handeln. Eine relative Menge reicht für die Bestimmung einer abnehmenden Bindungsrate aus. Wenn die Schritte a) bis c) bzw. a) bis d) mehrfach unter identischen Bedingungen durchgeführt werden, ist es ausreichend, wenn die Schritte a1) bis a3) zur Bestimmung der Zeit gemäß Schritt a3) nur einmal durchgeführt werden und die Zeit gemäß Schritt b) dann jeweils so gewählt ist, dass sie zumindest so lange ist, wie die bei Schritt a3) einmalig bestimmte Zeit.

Wie bei Bindungsversuchen üblich, werden die Antikörper, Antikörperfragmente oder Antikörpermimetika bei den Schritten a2) und b) jeweils in einer solchen Menge zugegeben, dass deren Konzentration in der Zellsuspension, der Teilmenge oder der weiteren Teilmenge die darin anzunehmende Konzentration an EpCAM oder sonstigem für epitheliale Zellen spezifischem Antigen bei weitem, mindestens jedoch um den Faktor 2, übersteigt.

Bei den Schritten b) und a2) kann nach Zugabe der Antikörper, Antikörperfragmente oder Antikörpermimetika und einer Inkubation für 5 bis 60 Minuten, beispielsweise nach 15 Minuten oder zwischen den Schritten b) und c) oder a2) und a3) eine Verdünnung der Suspension, beispielsweise mit PBS, erfolgen, um beim Bestimmen gemäß der Schritte c) und a3) bessere Messergebnisse zu erhalten, z. B. weil ein aus den nicht gebundenen Antikörpern, Antikörperfragmenten und Antikörpermimetika resultierendes Hintergrundsignal reduziert wird.

Durch das Bestimmen gemäß Schritt a3) wird die zur zuverlässigen Bestimmung einer Konzentration epithelialer Zellen erforderliche Inkubationszeit der Zellen mit den Antikörpern, Antikörperfragmenten oder Antikörpermimetika bestimmt. Diese Zeit kann zwischen 8 und 25 Stunden liegen. Bei Wahl einer verhältnismäßig hohen Temperatur kann eine abnehmende Bindungsrate jedoch auch bereits früher, z. B. nach 6 bis 8 Stunden, erreicht werden. Umgekehrt kann bei einer verhältnismäßig niedrigen Temperatur eine abnehmende Bindungsrate auch mehr als 25 Stunden erfordern und beispielsweise erst nach 25 bis 30 Stunden erreicht werden.

Die vorgegebene Temperatur kann eine Temperatur im Bereich von 0 bis 30 °C, insbesondere 0 bis 20 °C, insbesondere 8 bis 15 °C, sein.

Die Inkubation gemäß Schritt b) kann z. B. für mindestens 6 Stunden, mindestens 7 Stunden, mindestens 8 Stunden, mindestens 10 Stunden, mindestens 12 Stunden, mindestens 14 Stunden, mindestens 16 Stunden, mindestens 18 Stunden, mindestens 20 Stunden, mindestens 22 Stunden, mindestens 24 Stunden, mindestens 25 Stunden oder mindestens 30 Stunden erfolgen.

Die Markierung kann eine Fluoreszenzmarkierung mittels eines Fluorochroms, wie z. B. Fluoresceinisothiocyanat (FITC) oder Phycoerythrin (PE), eine Farbmarkierung mittels eines Chromophors oder eine indirekt detektierbare Markierung mittels Biotin, Avidin, Streptavidin oder einem sonstigen Affinitäts-Tag, Epitop-Tag oder Protein-Tag sein. Eine direkte Markierung des Antikörpers hat gegenüber einer indirekten Markierung, beispielsweise mittels eines Sekundärantikörpers, den Vorteil, dass ein Waschschritt, bei welchem üblicherweise ein Teil der epithelialen Zellen verloren geht, entfallen kann.

Das Bestimmen der Menge gebundener Antikörper, Antikörperfragmente oder Antikörpermimetika gemäß Schritt a3) und/oder das Bestimmen der Anzahl von durch eine Bindung der Antikörper, Antikörperfragmente oder Antikörpermimetika markierten Zellen gemäß Schritt c) kann mittels Laser-Scanning-Cytometrie, Fluoreszenzmikroskopie, insbesondere quantitativer Fluoreszenzmikroskopie, oder Lichtmikroskopie, insbesondere quantitativer Lichtmikroskopie, erfolgen. Wenn das Bestimmen der Menge gebundener Antikörper, Antikörperfragmente oder Antikörpermimetika und/oder das Bestimmen der Anzahl markierter Zellen mittels Fluoreszenzmikroskopie oder Lichtmikroskopie erfolgt, kann zur Erkennung der Zellen eine Software zur Bilderkennung eingesetzt werden. Diese kann markierte Zellen von markierten Zelltrümmern oder sonstigen unspezifisch markierten Strukturen unterscheiden.

Wenn die Markierung eine Fluoreszenzmarkierung ist, können bei der Laser-Scanning-Cytometrie oder der Fluoreszenzmikroskopie für jede der Zellen eine Gesamtfluoreszenz pro Zelle und eine Hintergrundfluoreszenz für diese Zelle dynamisch bestimmt werden, so dass für die Zellen äquivalente Fluoreszenzwerte jeweils als Differenz zwischen der für die jeweilige Zelle ermittelten Gesamtfluoreszenz und der für diese Zelle ermittelten Hintergrundfluoreszenz erhalten werden können. Bei der dynamischen Bestimmung der Hintergrundfluoreszenz wird jeweils bei der Messung der Gesamtfluoreszenz einer Zelle zum Zeitpunkt dieser Messung auch die Fluoreszenz des Hintergrunds im Bereich dieser Zelle, d. h. in einem Abstand bis zu etwa einem halben Zelldurchmesser von der Zellmembran dieser Zelle, gemessen. Dadurch können die Genauigkeit der jeweiligen Messung und die Aussagekraft der Messergebnisse erhöht werden.

Das Absondern der nicht lysierten Zellen gemäß Schritt a) kann durch Zentrifugation, bloße Sedimentation oder Filtration erfolgen. Das Suspendieren der abgesonderten Zellen in einem Puffer gemäß Schritt a) erfolgt unmittelbar nach dem Absondern, d. h. so, dass die Zellen nach dem Absondern nicht trocknen und durch das Trocknen beschädigt werden. Bei dem Puffer kann es sich um PBS, d. h. eine phosphatgepufferte Salzlösung, handeln.

Bei einer Ausgestaltung des Verfahrens wird zu der Zellsuspension, der Teilmenge oder der weiteren Teilmenge der Zellsuspension jeweils vor der Zugabe von je mindestens eine Markierung tragenden Antikörpern, Antikörperfragmenten oder Antikörpermimetika gemäß der Schritte b) und a2) ein Blockierungsreagenz zur Blockade unspezifischer Bindungsstellen und/oder von Fc-Rezeptoren zugesetzt und mit der Zellsuspension, der Teilmenge oder der weiteren Teilmenge der Zellsuspension vermischt und inkubiert. Das Inkubieren kann dabei beispielsweise für etwa 15 Minuten bei 8 °C erfolgen.

Bei einer Ausgestaltung des Verfahrens wird bei keinem der Schritte ein Detergens eingesetzt und/oder es erfolgt bei keinem der Schritte eine Fixierung der Zellen. Dadurch kann eine Schädigung der epithelialen Zellen und damit eine Beeinflussung des Messergebnisses vermieden werden. Dies ist insbesondere dann von Bedeutung, wenn bei dem Verfahren eine Konzentration von Zellen mit intakter Zellmembran oder sogar eine Konzentration lebender epithelialer Zellen bestimmt werden soll.

Sämtliche Schritte des Verfahrens oder zumindest die Schritte b) und c) oder zumindest die Schritte b), a2), a3) und c) können von einem dafür ausgelegten Automaten automatisiert durchgeführt werden. Dadurch können eine Objektivierung und Verbesserung der Reproduzierbarkeit der Messergebnisse erreicht werden.

Bei einer Ausgestaltung des Verfahrens werden bei Schritt a1) mehrere Teilmengen abgesondert, wobei bei Schritt a2) zu jeder der Teilmengen jeweils eine unterschiedliche Menge der Antikörper, Antikörperfragmente oder Antikörpermimetika zugegeben und, insbesondere sofort, mit der jeweiligen Teilmengen vermischt wird, wobei bei Schritt a3) für jede der Teilmengen die zum Erreichen der abnehmenden Bindungsrate erforderliche Zeit bestimmt wird, wobei für die Durchführung des Schritts b) dann diejenige Menge der Antikörper, Antikörperfragmente oder Antikörpermimetika gewählt wird, bei der die abnehmende Bindungsrate in einer gewünschten Zeit erreicht wird.

Das sonstige für epitheliale Zellen spezifische Antigen kann z. B. aus der folgenden Gruppe ausgewählt sein: Androgenrezeptor, Epithelial Growth Factor-Rezeptor (EGF-Rezeptor), Estrogenrezeptor, Progesteronrezeptor, Prostata spezifisches Antigen (PSA), Prostata spezifisches Membranantigen (PSMA), Programmed Death-Ligand 1 (PDL-1), Melan A, B7-H3, Vascular Endothelial Growth Factor-Rezeptor (VEGF-Rezeptor), Ki67, Insulin-like Growth Factor-Rezeptor (IGF-Rezeptor) und Her2neu.

Die Blutprobe oder die Aspiratsprobe kann für mindestens 14 Stunden, insbesondere mindestens 16 Stunden, insbesondere mindestens 18 Stunden, insbesondere mindestens 20 Stunden, insbesondere mindestens 22 Stunden, insbesondere mindestens 24 Stunden, bei einer Temperatur zwischen 0 und 40 °C, insbesondere bei einer Temperatur zwischen 15 und 25 °C, gelagert werden, bevor die Erythrozyten lysiert werden. Dadurch kann eine weitere Verbesserung der Zugänglichkeit der für epitheliale Zellen spezifischen Antigene erreicht werden.

Nachfolgend wird die Erfindung anhand eines Ausführungsbeispiels näher erläutert.

1 ml mit EDTA antikoaguliertes Blut wird für 24 Stunden bei Raumtemperatur stehen gelassen. Anschließend wird die Probe mit Erythrozytenlysepuffer (155 mM NH₄Cl, 10 mM KHCO₃, 1 mM Na₂-EDTA) auf 15 ml aufgefüllt und für 15 Minuten bei 8 °C gelagert. Anschließend wird die Probe bei 780 g für 7 Minuten zentrifugiert. Der Überstand wird vollständig verworfen. Das Pellet wird in 500 µl PBS-EDTA (137 mM NaCl, 2,8 mM KCl, 8,1 mM NaHPO₄, 1,47 mM KH₂PO₄, 2 mM EDTA, pH 7,4) resuspendiert.

Von der Probe werden 50 µl in ein Reaktionsgefäß gegeben und mit 15 µl FcR Blockierungsreagenz (Miltenyi Biotec GmbH) versetzt und für 15 Minuten bei 8 °C inkubiert. Anschließend werden 5 µl eines gegen EpCAM gerichteten monoklonalen Antikörpers (HEA-125, Miltenyi Biotec GmbH) hinzugegeben und mit der Suspension vermischt. Anschließend erfolgt eine 15-minütige lichtgeschützte Inkubation bei 8 °C. Danach werden 430 µl PBS-EDTA hinzugegeben und mit der Suspension vermischt. Die resultierende Suspension wird bei 8 °C im Dunkeln inkubiert. Nach erneutem Vermischen werden davon nach unterschiedlichen Zeiten jeweils 100 µl abgenommen und mittels eines Laser-Scanning-Mikroskops iCys (CompuCyte, Beckman Coulter GmbH) gemessen. Die Messung kann auch mittels eines anderen zur Fluoreszenzerfassung geeigneten Mikroskops erfolgen. Aus der gemessenen Bindung zu unterschiedlichen Zeiten kann die Zeit ab dem Vermischen mit dem Antikörper bestimmt werden, die zum Erreichen einer abnehmenden Bindungsrate benötigt wird.

Anschließend werden weitere 50 µl des in PBS-EDTA resuspendierten Zellpellets mit 15 µl FcR-Blockierungsreagenz versetzt und nach Vermischen für 15 Minuten bei 8 °C inkubiert. Anschließend werden 5 µl des oben genannten gegen EpCAM gerichteten Antikörpers hinzugegeben und mit der Zellsuspension vermischt. Nach 15 Minuten lichtgeschützter Inkubation bei 8 °C werden 430 µl PBS-EDTA hinzugegeben und mit der Suspension vermischt. Anschließend wird die Suspension für die zum Erreichen einer abnehmenden Bindungsrate erforderliche Zeit bei 8 °C im Dunkeln inkubiert. Nach einem erneuten gründlichen Mischen der Suspension werden 100 µl davon abgenommen und im Laser-Scanning-Mikroskop oder einem anderen zur Fluoreszenzerfassung geeigneten Mikroskop untersucht. Dabei wird die Anzahl der durch eine Bindung der Antikörper markierten Zellen bestimmt und daraus die Konzentration dieser Zellen in der ursprünglichen Blutprobe.

## Patentansprüche

1. Verfahren zur Bestimmung einer Konzentration epithelialer Zellen in einer aus einem Menschen oder Säugetier stammenden und mit einem gerinnungshemmenden Mittel versetzten Blutprobe oder Aspiratsprobe mit folgenden Schritten:
a) Lysieren der in der Blutprobe oder Aspiratsprobe enthaltenen Erythrozyten durch Zugabe eines ein Lysieren der Erythrozyten bewirkenden Puffers, Absondern dabei nicht lysierter Zellen und Suspendieren der abgesonderten Zellen in einem Puffer, wobei die Blutprobe oder die Aspiratsprobe für mindestens 24 Stunden bei einer Temperatur zwischen 0 und 40 °C gelagert wird, bevor die Erythrozyten lysiert werden,
b) Zugabe von je mindestens eine Markierung tragenden Antikörpern, Antikörperfragmenten oder Antikörpermimetika, die jeweils gegen das Epitheliale Zelladhäsionsmolekül (EpCAM) und/oder mindestens ein sonstiges für epitheliale Zellen spezifisches Antigen gerichtet sind, zu einer bei Schritt a) erhaltenen Zellsuspension oder einer durch Absondern erhaltenen Teilmenge dieser Zellsuspension, Vermischen der Antikörper, Antikörperfragmente oder Antikörpermimetika und der Zellsuspension oder der Teilmenge und Inkubation einer dadurch erhaltenen Mischung für mindestens eine Zeit, welche zum Erreichen einer abnehmenden Bindungsrate einer Bindung der Antikörper, Antikörperfragmente oder Antikörpermimetika an die Zellen erforderlich ist, wobei die Inkubation für mindestens 6 Stunden erfolgt,
c) Bestimmen der Anzahl von durch eine Bindung der Antikörper, Antikörperfragmente oder Antikörpermimetika markierten Zellen in der bei Schritt b) erhaltenen Mischung und
d) Berechnung der Konzentration der markierten Zellen, deren Anzahl bei Schritt c) bestimmt wurde, in der Blutprobe oder Aspiratsprobe.

2. Verfahren nach Anspruch 1, wobei zwischen den Schritten a) und b) die folgenden weiteren Schritte durchgeführt werden:
a1) Absondern mindestens einer Teilmenge oder einer weiteren Teilmenge der bei Schritt a) erhaltenen Zellsuspension,
a2) Zugabe der je mindestens eine Markierung tragenden Antikörper, Antikörperfragmente oder Antikörpermimetika, die jeweils gegen das Epitheliale Zelladhäsionsmolekül (EpCAM) und/oder mindestens ein sonstiges für epitheliale Zellen spezifisches Antigen gerichtet sind, zu der weiteren Teilmenge in einer solchen Menge, dass deren Konzentration in der weiteren Teilmenge mit der Konzentration der Antikörper, Antikörperfragmente oder Antikörpermimetika in der Zellsuspension oder der Teilmenge gemäß Schritt b) identisch ist, Vermischen der Antikörper, Antikörperfragmente oder Antikörpermimetika und der weiteren Teilmenge und Inkubation einer dadurch erhaltenen Mischung bei einer vorgegebenen Temperatur und
a3) fortlaufendes oder mehrfaches Bestimmen einer Menge von Antikörpern, Antikörperfragmenten oder Antikörpermimetika, die an in der weiteren Teilmenge enthaltene Zellen in Abhängigkeit von der Zeit ab dem Vermischen gemäß Schritt a2) gebunden sind, ermitteln einer Bindungsrate daraus und Bestimmen einer Zeit, die zum Erreichen einer abnehmenden Bindungsrate der Bindung der Antikörper, Antikörperfragmente oder Antikörpermimetika an diese Zellen erforderlich ist,
wobei die Inkubation bei Schritt b) bei der vorgegebenen Temperatur erfolgt, wobei die Zeit gemäß Schritt b) so gewählt ist, dass sie zumindest so lange ist, wie die bei Schritt a3) bestimmte Zeit.

3. Verfahren nach Anspruch 2, wobei die vorgegebene Temperatur eine Temperatur im Bereich von 0 bis 30 °C, insbesondere 0 bis 20 °C, insbesondere 8 bis 15 °C, ist.

4. Verfahren nach einem der vorhergehenden Ansprüche, wobei die Inkubation gemäß Schritt b) für mindestens 7 Stunden, mindestens 8 Stunden, mindestens 10 Stunden, mindestens 12 Stunden, mindestens 14 Stunden, mindestens 16 Stunden, mindestens 18 Stunden, mindestens 20 Stunden, mindestens 22 Stunden, mindestens 24 Stunden, mindestens 25 Stunden oder mindestens 30 Stunden erfolgt.

5. Verfahren nach einem der vorhergehenden Ansprüche, wobei die Markierung eine Fluoreszenzmarkierung mittels eines Fluorochroms, eine Farbmarkierung mittels eines Chromophors oder eine indirekt detektierbare Markierung mittels Biotin, Avidin, Streptavidin oder einem sonstigen Affinitäts-Tag, Epitop-Tag oder Protein-Tag ist.

6. Verfahren nach einem der vorhergehenden Ansprüche, wobei das Bestimmen der Menge gebundener Antikörper, Antikörperfragmente oder Antikörpermimetika gemäß Schritt a3) und/oder das Bestimmen der Anzahl von durch eine Bindung der Antikörper, Antikörperfragmente oder Antikörpermimetika markierten Zellen gemäß Schritt c) mittels Laser-Scanning-Cytometrie, Fluoreszenzmikroskopie oder Lichtmikroskopie erfolgt.

7. Verfahren nach Anspruch 6, wobei das Bestimmen der Menge gebundener Antikörper, Antikörperfragmente oder Antikörpermimetika und/oder das Bestimmen der Anzahl markierter Zellen mittels Fluoreszenzmikroskopie oder Lichtmikroskopie erfolgt und zur Erkennung der Zellen eine Software zur Bilderkennung eingesetzt wird.

8. Verfahren nach Anspruch 6 oder 7, wobei die Markierung eine Fluoreszenzmarkierung ist und bei der Laser-Scanning-Cytometrie oder der Fluoreszenzmikroskopie für jede der Zellen eine Gesamtfluoreszenz pro Zelle und eine Hintergrundfluoreszenz für diese Zelle dynamisch bestimmt werden, so dass für die Zellen äquivalente Fluoreszenzwerte jeweils als Differenz zwischen der für die jeweilige Zelle ermittelten Gesamtfluoreszenz und der für diese Zelle ermittelten Hintergrundfluoreszenz erhalten werden.

9. Verfahren nach einem der vorhergehenden Ansprüche, wobei das Absondern gemäß Schritt a) durch Zentrifugation, Sedimentation oder Filtration erfolgt.

10. Verfahren nach einem der vorhergehenden Ansprüche, wobei zu der Zellsuspension, der Teilmenge oder der weiteren Teilmenge der Zellsuspension jeweils vor der Zugabe von je mindestens eine Markierung tragenden Antikörpern, Antikörperfragmenten oder Antikörpermimetika gemäß der Schritte b) und a2) ein Blockierungsreagenz zur Blockade unspezifischer Bindungsstellen und/oder von Fc-Rezeptoren zugesetzt und mit der Zellsuspension, der Teilmenge oder der weiteren Teilmenge der Zellsuspension vermischt und inkubiert wird.

11. Verfahren nach einem der vorhergehenden Ansprüche, wobei bei keinem der Schritte ein Detergens eingesetzt wird und/oder bei keinem der Schritte eine Fixierung der Zellen erfolgt.

12. Verfahren nach einem der vorhergehenden Ansprüche, wobei sämtliche Schritte des Verfahrens oder zumindest die Schritte b) und c) oder zumindest die Schritte b), a2), a3) und c) von einem dafür ausgelegten Automaten automatisiert durchgeführt werden.

13. Verfahren nach einem der vorhergehenden Ansprüche, wobei bei Schritt a1) mehrere Teilmengen abgesondert werden, wobei bei Schritt a2) zu jeder der Teilmengen jeweils eine unterschiedliche Menge der Antikörper, Antikörperfragmente oder Antikörpermimetika zugegeben und mit der jeweiligen Teilmenge vermischt wird, wobei bei Schritt a3) für jede der Teilmengen die zum Erreichen der abnehmenden Bindungsrate erforderliche Zeit bestimmt wird, wobei für die Durchführung des Schritts b) dann diejenige Menge der Antikörper, Antikörperfragmente oder Antikörpermimetika gewählt wird, bei der die abnehmende Bindungsrate in einer gewünschten Zeit erreicht wird.

14. Verfahren nach einem der vorhergehenden Ansprüche, wobei das sonstige für epitheliale Zellen spezifische Antigen aus der folgenden Gruppe ausgewählt ist: Androgenrezeptor, Epithelial Growth Factor-Rezeptor (EGF-Rezeptor), Estrogenrezeptor, Progesteronrezeptor, Prostata spezifische Antigen (PSA), Prostata spezifisches Membranantigen (PSMA), Programmed Death-Ligand 1 (PDL-1), Melan A, B7-H3, Vascular Endothelial Growth Factor-Rezeptor (VEGF-Rezeptor), Ki67, Insulin-like Growth Factor-Rezeptor (IGF-Rezeptor) und Her2neu.

## Claims

1. A method for determining a concentration of epithelial cells in a blood sample or aspirate sample which originates from a human or mammal and has been admixed with an anticoagulant, comprising the following steps:
a) lysing the erythrocytes present in the blood sample or aspirate sample by addition of a buffer which brings about a lysis of the erythrocytes, segregating cells which are not lysed in the course of this, and suspending the segregated cells in a buffer, wherein the blood sample or the aspirate sample is stored at a temperature between 0 and 40°C for at least 24 hours before the erythrocytes are lysed,
b) adding antibodies, antibody fragments or antibody mimetics which each bear at least one label and which are each directed against the Epithelial Cell Adhesion Molecule (EpCAM) and/or at least one other antigen specific for epithelial cells to a cell suspension obtained in step a) or to a subquantity of said cell suspension, which subquantity has been obtained by segregation, mixing the antibodies, antibody fragments or antibody mimetics and the cell suspension or the subquantity, and incubating a mixture obtained as a result for at least a time which is required for the attainment of a decreasing binding rate of binding of the antibodies, antibody fragments or antibody mimetics to the cells, wherein the incubation is done for at least 6 hours,
c) determining the number of cells labeled by binding of the antibodies, antibody fragments or antibody mimetics, in the mixture obtained in step b), and
d) calculating the concentration of the labeled cells, the number of which has been determined in step c), in the blood sample or aspirate sample.

2. The method as claimed in claim 1, wherein the following further steps are carried out between steps a) and b):
a1) segregating at least one subquantity or a further subquantity of the cell suspension obtained in step a),
a2) adding the antibodies, antibody fragments or antibody mimetics which each bear at least one label and which are each directed against the Epithelial Cell Adhesion Molecule (EpCAM) and/or at least one other antigen specific for epithelial cells to the further subquantity in such a quantity that the concentration thereof in the further subquantity is identical to the concentration of the antibodies, antibody fragments or antibody mimetics in the cell suspension or the subquantity according to step b), mixing the antibodies, antibody fragments or antibody mimetics and the further subquantity, and incubating a mixture obtained as a result at a predefined temperature and
a3) continuously or repeatedly determining a quantity of antibodies, antibody fragments or antibody mimetics which have bound to cells present in the further subquantity as a function of time starting from the mixing according to step a2), ascertaining a binding rate therefrom, and determining a time which is required for the attainment of a decreasing binding rate of the binding of the antibodies, antibody fragments or antibody mimetics to said cells,
wherein the incubation in step b) is done at the predefined temperature, wherein the time according to step b) is selected such that it is at least as long as the time determined in step a3).

3. The method as claimed in claim 2, wherein the predefined temperature is a temperature in the range from 0 to 30°C, especially 0 to 20°C, especially 8 to 15°C.

4. The method as claimed in any of the preceding claims, wherein the incubation according to step b) is done for at least 7 hours, at least 8 hours, at least 10 hours, at least 12 hours, at least 14 hours, at least 16 hours, at least 18 hours, at least 20 hours, at least 22 hours, at least 24 hours, at least 25 hours, or at least 30 hours.

5. The method as claimed in any of the preceding claims, wherein the label is a fluorescent label by means of a fluorochrome, a color label by means of a chromophore, or an indirectly detectable label by means of biotin, avidin, streptavidin or some other affinity tag, epitope tag or protein tag.

6. The method as claimed in any of the preceding claims, wherein the determination of the quantity of bound antibodies, antibody fragments or antibody mimetics according to step a3) and/or the determination of the number of cells labeled by binding of the antibodies, antibody fragments or antibody mimetics according to step c) is done by means of laser scanning cytometry, fluorescence microscopy or light microscopy.

7. The method as claimed in claim 6, wherein the determination of the quantity of bound antibodies, antibody fragments or antibody mimetics and/or the determination of the number of labeled cells is done by means of fluorescence microscopy or light microscopy and an image recognition software is used to identify the cells.

8. The method as claimed in claim 6 or 7, wherein the label is a fluorescent label and what are dynamically determined in the laser scanning cytometry or the fluorescence microscopy for each of the cells are a total fluorescence per cell and a background fluorescence for said cell, such that equivalent fluorescence values are obtained for the cells, in each case as the difference between the total fluorescence ascertained for the particular cell and the background fluorescence ascertained for said cell.

9. The method as claimed in any of the preceding claims, wherein the segregation according to step a) is done by centrifugation, sedimentation or filtration.

10. The method as claimed in any of the preceding claims, wherein a blocking reagent for the blocking of nonspecific binding sites and/or of Fc receptors is added to the cell suspension, the subquantity or the further subquantity of the cell suspension and mixed and incubated with the cell suspension, the subquantity or the further subquantity of the cell suspension, in each case before the addition of antibodies, antibody fragments or antibody mimetics which each bear at least one label according to steps b) and a2).

11. The method as claimed in any of the preceding claims, wherein in none of the steps is a detergent used and/or in none of the steps are the cells fixed.

12. The method as claimed in any of the preceding claims, wherein all the steps of the method or at least steps b) and c) or at least steps b), a2), a3) and c) are carried out in an automated manner by a machine designed for this purpose.

13. The method as claimed in any of the preceding claims, wherein two or more subquantities are segregated in step a1), wherein, in step a2), a different quantity of the antibodies, antibody fragments or antibody mimetics is added in each case to each of the subquantities and mixed with the respective subquantity, wherein, in step a3), the time required for the attainment of the decreasing binding rate is determined for each of the subquantities, wherein that quantity of the antibodies, antibody fragments or antibody mimetics in which the decreasing binding rate is attained within a desired time is then selected for the performance of step b).

14. The method as claimed in any of the preceding claims, wherein the other antigen specific for epithelial cells is selected from the following group: androgen receptor, epithelial growth factor receptor (EGF receptor), estrogen receptor, progesterone receptor, prostate-specific antigen (PSA), prostate-specific membrane antigen (PSMA), programmed death ligand 1 (PDL-1), melan A, B7-H3, vascular endothelial growth factor receptor (VEGF receptor), Ki67, insulin-like growth factor receptor (IGF receptor) and Her2neu.

## Revendications

1. Procédé pour la détermination d'une concentration en cellules épithéliales dans un échantillon de sang ou un échantillon d'aspirat provenant d'un humain ou d'un mammifère et mélangé à un agent anticoagulant comprenant les étapes suivantes consistant à :
a) lyser les érythrocytes contenus dans l'échantillon de sang ou l'échantillon d'aspirat par addition d'un tampon provoquant une lyse des érythrocytes, y séparer des cellules non lysées, et tenir en suspension les cellules séparées dans un tampon, en ce que l'échantillon de sang ou l'échantillon d'aspirat est conservé durant au moins 24 heures à une température comprise entre 0 et 40 °C, avant la lyse des érythrocytes,
b) ajouter des anticorps, des fragments d'anticorps ou des mimétiques d'anticorps portant chacun au moins un marquage, qui sont respectivement dirigés contre la molécule d'adhérence cellulaire épithéliale (EpCAM) et/ou au moins contre un autre antigène spécifique aux cellules épithéliales, à une suspension cellulaire obtenue à l'étape a) ou à une partie obtenue par séparation de cette suspension cellulaire, mélanger les anticorps, fragments d'anticorps ou mimétiques d'anticorps et la suspension cellulaire ou la partie et incuber un mélange ainsi obtenu pour au moins une durée qui est nécessaire à l'obtention d'un taux de liaison décroissant d'une liaison des anticorps, fragments d'anticorps ou mimétiques d'anticorps aux cellules, en ce que l'incubation se produit durant au moins 6 heures,
c) déterminer le nombre de cellules marquées par une liaison des anticorps, fragments d'anticorps ou mimétiques d'anticorps dans le mélange obtenu à l'étape b) et
d) calculer la concentration des cellules marquées, dont le nombre a été déterminé à l'étape c), dans l'échantillon de sang ou l'échantillon d'aspirat.

2. Procédé selon la revendication 1, en ce que les autres étapes suivantes entre les étapes a) et b) sont effectuées :
a1) séparation d'au moins une partie ou d'une autre partie de la suspension cellulaire obtenue à l'étape a),
a2) addition des anticorps, fragments d'anticorps ou mimétiques d'anticorps portant chacun au moins un marquage, qui sont respectivement dirigés contre la molécule d'adhérence cellulaire épithéliale (EpCAM) et/ou au moins contre un autre antigène spécifique aux cellules épithéliales, à l'autre partie dans une telle quantité que sa concentration dans l'autre partie est identique à la concentration des anticorps, fragments d'anticorps ou mimétiques d'anticorps dans la suspension cellulaire ou la partie selon l'étape b), mélange des anticorps, fragments d'anticorps ou mimétiques d'anticorps et de l'autre partie et incubation d'un mélange ainsi obtenu à une température prédéfinie et
a3) détermination continue ou multiple d'une quantité d'anticorps, de fragments d'anticorps ou mimétiques d'anticorps, qui sont liés aux cellules contenues dans l'autre partie en fonction de la durée à partir du mélange selon l'étape a2), détermination à partir de cela d'un taux de liaison et définition d'une durée qui est nécessaire à l'obtention d'un taux de liaison décroissant de la liaison des anticorps, fragments d'anticorps ou mimétiques d'anticorps à ces cellules,
en ce que l'incubation à l'étape b) se produit à la température prédéfinie, en ce que la durée selon l'étape b) est sélectionnée de façon à ce qu'elle soit au moins aussi longue que la durée définie à l'étape a3).

3. Procédé selon la revendication 2, en ce que la température prédéfinie est une température dans une plage comprise entre 0 et 30 °C, en particulier entre 0 et 20 °C, en particulier entre 8 et 15 °C.

4. Procédé selon l'une des revendications précédentes, en ce que l'incubation selon l'étape b) se produit durant au moins 7 heures, au moins 8 heures, au moins 10 heures, au moins 12 heures, au moins 14 heures, au moins 16 heures, au moins 18 heures, au moins 20 heures, au moins 22 heures, au moins 24 heures, au moins 25 heures ou au moins 30 heures.

5. Procédé selon l'une des revendications précédentes, en ce que le marquage est un marquage fluorescent au moyen d'un fluorochrome, un marquage en couleur au moyen d'un chromophore ou un marquage indirectement détectable au moyen de biotine, d'avidine, de streptavidine ou d'une autre étiquette d'affinité, étiquette d'épitope ou étiquette de protéine.

6. Procédé selon l'une des revendications précédentes, en ce que la détermination de la quantité d'anticorps, de fragments d'anticorps ou mimétiques d'anticorps liés selon l'étape a3) et/ou la détermination du nombre de cellules marquées selon l'étape c) par une liaison des anticorps, fragments d'anticorps ou mimétiques d'anticorps s'effectue au moyen de la cytométrie à balayage laser, de la microscopie à fluorescence ou de la microscopie optique.

7. Procédé selon la revendication 6, en ce que la détermination de la quantité d'anticorps, de fragments d'anticorps ou de mimétiques d'anticorps liés et/ou la détermination du nombre de cellules marquées s'effectue au moyen de la microscopie à fluorescence ou de la microscopie optique et qu'un logiciel d'analyse d'image est utilisé pour l'identification des cellules.

8. Procédé selon la revendication 6 ou 7, en ce que le marquage est un marquage fluorescent et que, dans le cas de la cytométrie à balayage laser ou de la microscopie à fluorescence pour chacune des cellules, une fluorescence totale par cellule et une fluorescence en arrière-plan pour cette cellule sont déterminées dynamiquement, de sorte que pour les cellules des valeurs de fluorescence équivalentes sont obtenues respectivement en tant que différence entre la fluorescence totale déterminée pour la cellule respective et la fluorescence en arrière-plan déterminée pour cette cellule.

9. Procédé selon l'une des revendications précédentes, en ce que la séparation selon l'étape a) s'effectue par centrifugation, sédimentation ou filtration.

10. Procédé selon l'une des revendications précédentes, en ce qu'un réactif de blocage pour le blocage de sites de liaison non spécifiques et/ou de récepteurs Fc est ajouté à la suspension cellulaire, à la partie ou l'autre partie de la suspension cellulaire respectivement avant l'addition d'anticorps, de fragments d'anticorps ou de mimétiques d'anticorps portant chacun au moins un marquage selon les étapes b) et a2) et est mélangé à la suspension cellulaire, à la partie ou l'autre partie de la suspension cellulaire puis incubé.

11. Procédé selon l'une des revendications précédentes, en ce qu'un détergent n'est utilisé dans aucune des étapes et/ou une fixation des cellules n'est effectuée dans aucune des étapes.

12. Procédé selon l'une des revendications précédentes, en ce que toutes les étapes du procédé ou au moins les étapes b) et c) ou au moins les étapes b), a2), a3) et c) sont effectuées par une machine automatique dimensionnée à cet effet.

13. Procédé selon l'une des revendications précédentes, en ce que plusieurs parties sont séparées à l'étape a1), en ce qu'à l'étape a2) respectivement une quantité différente d'anticorps, de fragments d'anticorps ou de mimétiques d'anticorps est ajoutée à chacune des parties et mélangée à la partie respective, en ce que la durée nécessaire à l'obtention du taux de liaison décroissant pour chacune des parties est déterminée à l'étape a3), puis en ce que pour l'exécution de l'étape b) la quantité d'anticorps, de fragments d'anticorps ou de mimétiques d'anticorps est sélectionnée parmi laquelle le taux de liaison décroissant est obtenu dans une durée souhaitée.

14. Procédé selon l'une des revendications précédentes, en ce que l'autre antigène spécifique aux cellules épithéliales est sélectionné parmi le groupe suivant : récepteur androgène, récepteur de facteur de croissance épithélial (récepteur EGF), récepteur des oestrogènes, récepteur de progestérone, antigène prostatique spécifique (PSA), antigène membranaire prostatique spécifique (PSMA), ligand de mort cellulaire programmée 1 (PDL-1), Melan A, B7-H3, récepteur aux facteurs de croissance endothéliale vasculaire (récepteur VEGF) Ki67, récepteur au facteur de croissance de type insuline (récepteur IGF) et Her2neu.
